# EUROPEAN PATENT APPLICATION

(11) **EP 4 520 371 A1**
(43) Date of publication of application: **12.03.2025**
(21) Application number: 23865651.6
(22) Date of filing: 02.06.2023
(51) Int. Cl.: A61M 15/00, A24F 40/20, A24F 40/42, A24F 40/40

(54) **INHALER**

(30) Priority: 14.09.2022 KR 20220115619
(71) Applicant: KT&G Corporation, Daedeok-gu Daejeon 34337 (KR)
(72) Inventor: JUNG, Yongmi, Daejeon 34128 (KR); KIM, Moonwon, Daejeon 34128 (KR); SHIN, Jun Won, Daejeon 34128 (KR)
(74) Representative: Ter Meer Steinmeister & Partner
(86) International application number: PCT/KR2023/007614
(87) International publication number: WO 2024/058347

(57) **Abstract**

An inhaler for inhaling a functional material according to various embodiments comprises: a holder that includes an opening and an inner space communicating with the opening; piercing members which protrude into the inner space; and a functional material-accommodating member which is inserted along the longitudinal axis of the holder into the inner space of the holder from the opening and accommodates the functional material. Each of the piercing members can move between a first piercing position and a second piercing position, and the tip of each of the piercing members may be closer to the longitudinal axis when the piercing member is at the second piercing position than when the piercing member is at the first piercing position.

## Description

### TECHNICAL FIELD

The following embodiments relate to an inhaler.

### BACKGROUND ART

Research has been conducted on an inhaler that may deliver a target substance directly to the lungs of a user. For example, Korean Patent Publication No. 10-2016-0065204 discloses a dry powder inhaler.

### DISCLOSURE OF THE INVENTION

### TECHNICAL GOALS

An inhaler according to various embodiments may induce a vortex behavior of functional substance powder.

An inhaler according to various embodiments may induce smooth discharge of functional substance powder.

### TECHNICAL SOLUTIONS

An inhaler according to an embodiment may include a holder including an opening and an internal space that communicates with the opening, a piercing member protruding toward the internal space, and a functional substance accommodation member inserted from the opening into the internal space of the holder along a longitudinal axis of the holder and configured to accommodate the functional substance, wherein the piercing member may be configured to be movable between a first piercing position and a second piercing position, and wherein a cutting edge of the piercing member may be closer to the longitudinal axis in the second piercing position than in the first piercing position.

According to an embodiment, the piercing member may include a first piercing member and a second piercing member disposed spaced apart from the first piercing member along the longitudinal axis.

According to an embodiment, the first piercing member and the second piercing member may be disposed to face each other about the longitudinal axis.

According to an embodiment, a cutting edge of the first piercing member and a cutting edge of the second piercing member may be directed toward the longitudinal axis of the holder.

According to an embodiment, the piercing member may be configured to be capable of protruding in a direction perpendicular to the longitudinal axis, the first piercing position may be a state in which the piercing member returns to an initial position, and the second piercing position may be a state in which the piercing member protrudes toward the longitudinal axis.

According to an embodiment, the piercing member may be pivotally rotatably coupled to the holder, the inhaler may further include a stopping member in contact with the piercing member and configured to prevent a rotation of the piercing member, and the stopping member may be configured to prevent the piercing member from rotating toward the opening. According to an embodiment, the first piercing position may be a state in which a cutting edge of the piercing member is rotated toward an opposite side of the opening, and the second piercing position may be a state in which a cutting edge of the piercing member is rotated toward the longitudinal axis.

According to an embodiment, a marker for indicating a length at which the functional substance accommodation member is inserted into the internal space may be provided in at least one of the functional substance accommodation member or the holder.

According to an embodiment, the functional substance accommodation member may include a stick insertable into an internal space of the holder and a capsule disposed in an inside of the stick and configured to accommodate the functional substance.

### EFFECTS OF THE INVENTION

According to various embodiments, a vortex behavior of functional substance powder may be induced.

According to various embodiments, smooth discharge of functional substance powder may be performed by internal airflow introduced into an inhaler penetrating a center of a functional substance accommodation member.

The effects of an inhaler according to various embodiments are not limited to the above-mentioned effects, and other unmentioned effects can be clearly understood from the following description by one of ordinary skill in the art to which the present disclosure pertains.

### BRIEF DESCRIPTION OF DRAWINGS

FIG. 1 is a diagram illustrating an inhaler according to various embodiments.
FIGs. 2A to 2C are diagrams illustrating an operating state of an inhaler according to various embodiments.
FIG. 3 is a diagram illustrating an inhaler according to various embodiments.
FIGs. 4A to 4C are diagrams illustrating an operating state of an inhaler according to various embodiments.

### BEST MODE FOR CARRYING OUT THE INVENTION

The terms used in the embodiments are selected from among common terms that are currently widely used, in consideration of their function in the embodiments. However, the terms may become different according to an intention of one of ordinary skill in the art, a precedent, or the advent of new technology. Also, in particular cases, the terms are discretionally selected by the applicant of the disclosure, and the meaning of those terms will be described in detail in the corresponding part of the detailed description. Therefore, the terms used in the disclosure are not merely designations of the terms, but the terms are defined based on the meaning of the terms and content throughout the disclosure.

It will be understood that when a certain part "includes" a certain component, the part does not exclude another component but may further include another component, unless the context clearly dictates otherwise. Also, terms such as "unit," "module," etc., as used in the specification may refer to a part for processing at least one function or operation and may be implemented as hardware, software, or a combination of hardware and software.

As used herein, an expression such as "at least one of" that precedes listed components modifies not each of the listed components but all the components. For example, expressions "at least one of a, b, or c" and "at least one of a, b, and c" should be construed as referring to "a," "b," "c," "a and b," "a and c," "b and c," or "a, b, and c."

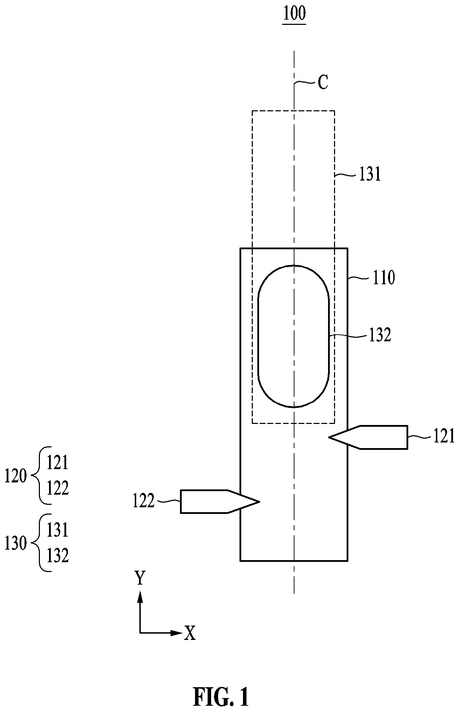

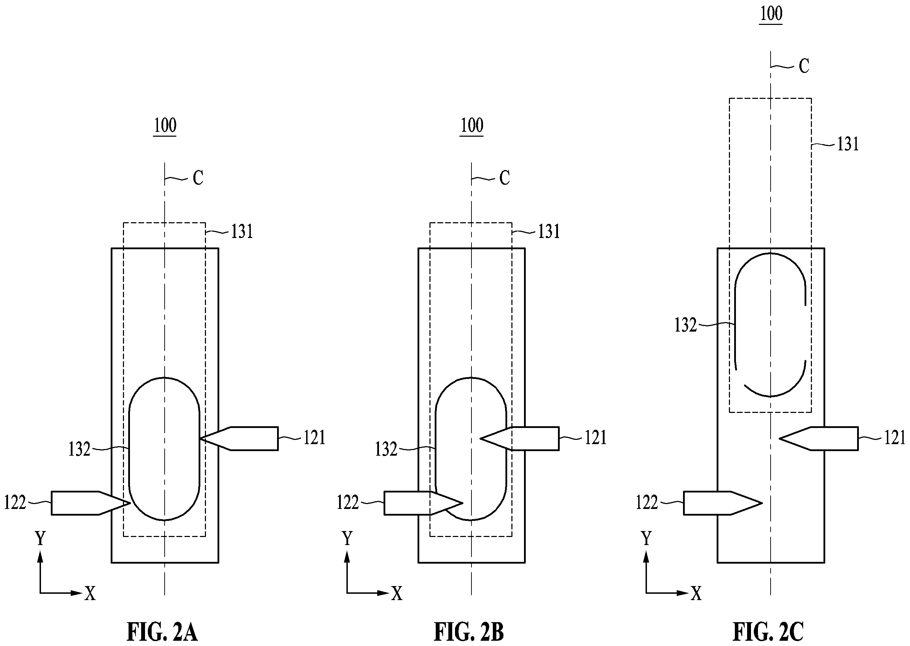

FIG. 1 is a diagram illustrating an inhaler 100 according to various embodiments, and FIGs. 2A to 2C are diagrams illustrating an operating state of the inhaler 100 according to various embodiments.

The inhaler 100 according to an embodiment may deliver a functional substance to a user. For example, the inhaler 100 according to an embodiment may be composed of an inhaler that delivers a dried pharmacological substance or nicotine to the lungs of a user in a form of an aerosol.

Referring to FIG. 1, the inhaler 100 according to an embodiment may include a holder 110, a piercing member 120, and a functional substance accommodation member 130.

In an embodiment, the holder 110 may be configured in a cylindrical shape with an internal space, and the internal space may be formed to a size into which the functional substance accommodation member 130, which is described below, may be inserted. An opening may be formed on a side of the holder 110, wherein the functional substance accommodation member 130 is inserted through the opening, and the opening and the internal space may be connected to each other.

In an embodiment, the piercing member 120 may include a first piercing member 121 and a second piercing member 122. One side of the first piercing member 121 may be disposed inside the holder 110, and the other side of the first piercing member 121 may be configured as a cutting edge protruding toward a longitudinal axis C of the holder 110. One side of the second piercing member 122 may be disposed inside the holder 110, and the other side of the second piercing member 122 may be configured as a cutting edge protruding toward the longitudinal axis C of the holder 110.

The first piercing member 121 and the second piercing member 122 may be disposed to be spaced apart in a direction parallel to the longitudinal axis C. For example, the first piercing member 121 and the second piercing member 122 may be disposed to be spaced apart from each other in a ±Y direction of FIG. 1. In addition, the first piercing member 121 and the second piercing member 122 may be disposed to face each other about the longitudinal axis C. For example, the first piercing member 121 and the second piercing member 122 may be disposed to be spaced apart from each other in a ±X direction of FIG. 1 with respect to the longitudinal axis C.

In an embodiment, the functional substance accommodation member 130 may include a stick 131 and a capsule 132. For example, the stick 131 may be configured in a cylindrical shape that may be inserted into the internal space of the holder 110, and the capsule 132 may be disposed in an inside of the stick 131.

An area of the functional substance accommodation member 130, in which the capsule 132 may be disposed, may be set so that the capsule 132 may be in a position to face the first piercing member 121 or the second piercing member 122 when the functional substance accommodation member 130 is inserted in the internal space of the holder 110.

In an example, the capsule 132 may accommodate a functional substance. The functional substance may include, for example, at least one of nicotine, theanine, caffeine, taurine, a pharmacological substance, or a mixture thereof. The functional substance may be in a form of fine granules or dry powder.

In an example, an outer shell of the capsule 132 may be made of a material that may be pierced or cut by the first piercing member 121 or the second piercing member 122.

In an embodiment, the first piercing member 121 and the second piercing member 122 may move between a first piercing position (e.g., a position of the first piercing member 121 and the second piercing member 122 of FIG. 2A) and a second piercing position (e.g., a position of the first piercing member 121 and the second piercing member 122 of FIG. 2B), and a cutting edge of the piercing member 120 may be closer to the longitudinal axis C in the second piercing position than in the first piercing position.

Here, a horizontal distance (e.g., a ±X direction distance of FIG. 1) between the first piercing member 121 and the second piercing member 122 in the first piercing position may be set to be greater than an outer diameter of the functional substance accommodation member 130, and the horizontal distance (e.g., the ±X direction distance of FIG. 1) between the first piercing member 121 and the second piercing member 122 in the second piercing position may be set to be less than an outer diameter of the capsule 132.

For example, before the functional substance accommodation member 130 is inserted into the internal space of the holder 110, the first piercing member 121 and the second piercing member 122 may be positioned in the first piercing position. Thereafter, the functional substance accommodation member 130 may be inserted into the internal space of the holder 110 without being disturbed by the first piercing member 121 or the second piercing member 122. In a state in which the functional substance accommodation member 130 is inserted into the internal space of the holder 110, the first piercing member 121 and the second piercing member 122 may be in the second piercing position. In the second piercing position, the first piercing member 121 or the second piercing member 122 may penetrate the capsule 132 so that at least a portion of the capsule 132 may be open and the functional substance in the capsule 132 may be exposed to an outside of the capsule 142.

In an embodiment, the first piercing member 121 or the second piercing member 122 may be formed to be capable of protruding in a direction (e.g., the ±X direction of FIG. 1) perpendicular to the longitudinal axis C. Here, the first piercing position may be a state in which the first piercing member 121 or the second piercing member 122 returned to an initial position, and the second piercing position may be a state in which the first piercing member 121 or the second piercing member 122 protrudes toward the longitudinal axis C. For example, an elastic member for helping the first piercing member 121 or the second piercing member 122 protrude or return to the initial position may be provided.

In an embodiment, a marker (not shown) for indicating a length at which the stick 131 is inserted into the internal space may be provided in at least one of the holder 110 or the stick 131 of the functional substance accommodation member 130. When an open area in a longitudinal slit shape is formed in the capsule 132, a size of the slit may be indirectly measured by the marker, and the size of the slit may be set to be proportional to an amount of a puff of the functional substance.

Hereinafter, the operating state of the inhaler 100 according to various embodiments is described with reference to FIGs. 2A to 2C.

Referring to FIG. 2A, the functional substance accommodation member 130 may be inserted into the internal space of the holder 110. Here, the first piercing member 121 or the second piercing member 122 may be in the first piercing position.

Referring to FIGS. 2A and 2B, the first piercing member 121 or the second piercing member 122 may move from the first piercing position to the second piercing position, and the capsule 132 may be penetrated by the first piercing member 121 or the second piercing member 122.

When the first piercing member 121 or the second piercing member 122 returns to the first piercing position, a penetrating hole may be formed in the capsule 132.

Alternatively, the first piercing member 121 or the second piercing member 122 may separate the stick 131 from the internal space of the holder 110 while the first piercing member 121 or the second piercing member 122 is inserted in the capsule 132, and in this case, a longitudinal slit may be formed in the capsule 132.

A penetrating hole or a slit-shaped open area due to the first piercing member 121, or a penetrating hole or a slit-shaped open area due to the second piercing member 122, may be formed in the capsule 132. Since the open areas may be formed spaced apart along the longitudinal axis C, airflow passing through the open areas may pass a central area of the capsule 132.

Referring to FIG. 2C, at least a portion of the functional substance accommodation member 130 may be separated from the holder 110. Here, the functional substance accommodation member 130 may be completely separated from the holder 110, but the functional substance may transfer while the capsule 132 is inserted in the holder 110. In this case, separation of the functional substance may effectively be prevented compared to a case in which the functional substance accommodation member 130 is completely separated from the holder 110, and thus, unnecessary leakage of the functional substance may be prevented.

When the user bites one end of the stick 131 and makes an inhaling motion while the open area is formed in the capsule 132, external air may flow into the internal space of the holder 110 through an airflow hole (not shown) formed in the holder 110, and the air may flow into the capsule 132 through the open area formed by the second piercing member 122. Subsequently, the air may escape through the open area formed by the first piercing member 121 and reach the user, in which case the functional substance in the capsule 132 may also be transferred to the user. Due to eccentric positions of the open areas, the air may pass a central part of the capsule 132 and, at the same time, a vortex behavior of the functional substance may be induced. The vortex behavior may induce smooth movement of the functional substance, and accordingly, discharge of the functional substance may be performed more smoothly.

In an example, when the capsule 132 is disposed in the stick 131 in an unfixed state, air movement (airflow) generated due to an eccentric position of the open area may induce the rotation of the capsule 132. Through this, the discharge of the functional substance may be performed more smoothly.

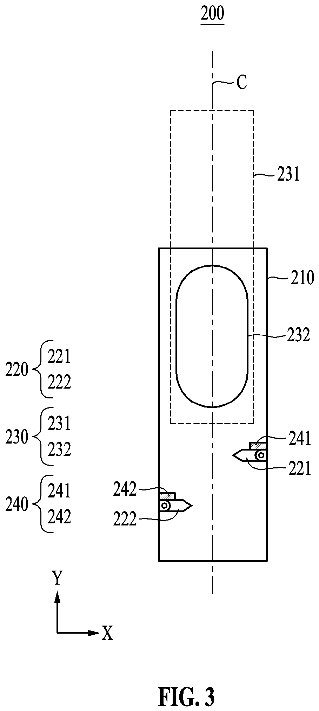

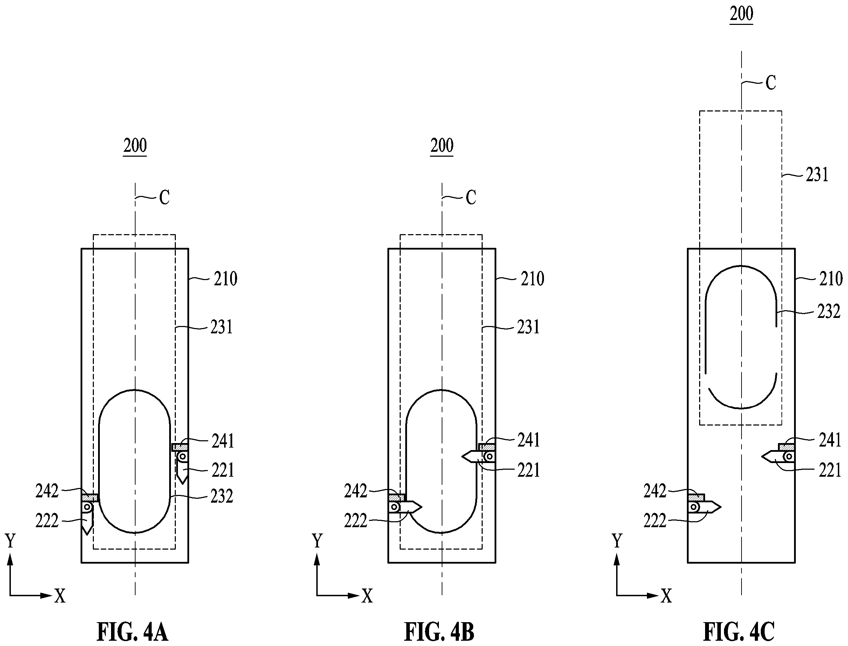

FIG. 3 is a diagram illustrating an inhaler 200 according to various embodiments, and FIGs. 4A to 4C are diagrams illustrating an operating state of the inhaler 200 according to various embodiments.

Referring to FIG. 3, the inhaler 200 according to an embodiment may include a holder 210, a piercing member 220, and a functional substance accommodation member 230. A structure and functions of the holder 210 may be identical or similar to those of the holder 110 described above, and a structure and functions of the functional substance accommodation member 230 including a stick 231 and a capsule 232 may be identical or similar to those of the functional substance accommodation member 130 described above, and descriptions thereof are thus omitted for simplicity.

In an embodiment, the piercing member 220 may include a first piercing member 221 and a second piercing member 222. One side of the first piercing member 221 may be pivotally rotatably connected to an inside of the holder 210, and the other side of the first piercing member 221 may be configured as a cutting edge. One side of the second piercing member 222 may be pivotally rotatably connected to the inside of the holder 210, and the other side of the second piercing member 222 may be configured as a cutting edge.

The first piercing member 221 and the second piercing member 222 may be disposed to be spaced apart in a direction parallel to the longitudinal axis C. For example, the first piercing member 221 and the second piercing member 222 may be disposed to be spaced apart from each other in a ±Y direction of FIG. 3. In addition, the first piercing member 221 and the second piercing member 222 may be disposed to face each other about the longitudinal axis C. For example, the first piercing member 221 and the second piercing member 222 may be disposed to be spaced apart from each other in a ±X direction of FIG. 3 with respect to the longitudinal axis C.

In an embodiment, the first piercing member 221 and the second piercing member 222 may move between a first piercing position (e.g., a position of the first piercing member 221 and the second piercing member 222 of FIG. 4A) and a second piercing position (e.g., a position of the first piercing member 221 and the second piercing member 222 of FIG. 4B), and the cutting edge of the first piercing member 221 or the cutting edge of the second piercing member 222 may be closer to the longitudinal axis C in the second piercing position than in the first piercing position.

Here, a horizontal distance (e.g., a ±X direction distance of FIG. 3) between the cutting edge of the first piercing member 221 and the cutting edge of the second piercing member 222 in the first piercing position may be set to be greater than an outer diameter of the functional substance accommodation member 230, and the horizontal distance (e.g., the ±X direction distance of FIG. 3) between the cutting edge of the first piercing member 221 and the cutting edge of the second piercing member 222 in the second piercing position may be set to be less than an outer diameter of the capsule 232.

For example, before the functional substance accommodation member 230 is inserted into the internal space of the holder 210, the first piercing member 221 and the second piercing member 222 may be positioned in the first piercing position. Thereafter, the functional substance accommodation member 230 may be inserted into the internal space of the holder 210 without being disturbed by the first piercing member 221 or the second piercing member 222. **In** a state in which the functional substance accommodation member 230 is inserted into the internal space of the holder 210, the first piercing member 221 and the second piercing member 222 may be in the second piercing position. In the second piercing position, the first piercing member 221 or the second piercing member 222 may penetrate the capsule 232 so that at least a portion of the capsule 232 may be open and the functional substance in the capsule 232 may be exposed to an outside of the capsule 232.

In an embodiment, the first piercing member 221 or the second piercing member 222 may be pivotally rotatably coupled to the holder 210. For example, the first piercing member 221 or the second piercing member 222 may be coupled to the inside of the holder 210 by a hinge coupling method, and the first piercing member 221 or the second piercing member 222 may rotate between the first piercing position, in which the cutting edges of the first piercing member 221 and the second piercing member 222 are rotated toward an opposite side of the opening of the holder 210, and the second piercing position, in which the cutting edges of the first piercing member 221 and the second piercing member 222 are directed toward the longitudinal axis C.

In an embodiment, the inhaler 200 according to an embodiment may further include a stopping member 240 that may contact the piercing member 220 and prevent a rotation of the piercing member 220. The stopping member 240 may prevent the piercing member 220 from rotating toward the opening, thereby allowing the piercing member 220 to rotate only between the first piercing position and the second piercing position. A slit-shaped open area may be formed in the capsule 232 by the stopping member 240.

In an example, the stopping member 240 may include a first stopping member 241 in contact with the first piercing member 221 and a second stopping member 242 in contact with the second piercing member 222. For example, the first stopping member 241 may be located between the first piercing member 221 and the opening and be in contact with the first piercing member 221 to prevent the cutting edge of the first piercing member 221 from rotating toward the opening. Similarly, the second stopping member 242 may be located between the second piercing member 222 and the opening and be in contact with the second piercing member 222 to prevent the cutting edge of the second piercing member 222 from rotating toward the opening.

In an embodiment, the inhaler 200 according to an embodiment may further include an elastic element that may rotate the piercing member 220 toward the second piercing position. For example, the elastic element may be a coil spring, and the coil spring may connect the stopping member 240 and the piercing member 220 together and return the piercing member 220, which is rotated to the first piercing position, to the second piercing position.

In an embodiment, a marker (not shown) for indicating a length at which the stick 231 is inserted into the internal space may be provided in at least one of the holder 210 or the stick 231 of the functional substance accommodation member 230. A size of a longitudinal slit formed in the capsule 232 may be indirectly measured by the marker, and the size of the slit may be set to be proportional to an amount of a puff of the functional substance.

Hereinafter, the operating state of the inhaler 200 according to an embodiment is described with reference to FIGs. 4A to 4C.

Referring to FIG. 4A, when the functional substance accommodation member 230 is inserted into the internal space through the opening of the holder 210, the first piercing member 221 or the second piercing member 222 may rotate so that the cutting edges may be directed toward the opposite of the opening (i.e., in a state of the first piercing position). Since the first piercing member 221 and the second piercing member 222 may rotate without hindrance in a direction opposite to the opening, the functional substance accommodation member 230 may be inserted into the holder 210 without being disturbed by the first piercing member 221 or the second piercing member 222.

Referring to FIG. 4B, when the functional substance accommodation member 230 begins to be pulled toward an outside of the holder 210, the first piercing member 221 or the second piercing member 222 may rotate so that the cutting edges may be directed toward the longitudinal axis C (i.e., in a state of the second piercing position). Here, the cutting edges of the first piercing member 221 or the second piercing member 222 may penetrate the capsule 232. When the functional substance accommodation member 230 continues to be pulled toward the outside of the holder 210, longitudinal slit-shaped open areas may be formed in the capsule 232 by the first piercing member 221 or the second piercing member 222.

Here, a slit by the first piercing member 221 and a slit by the second piercing member 222 may be formed, respectively. Since the slits may be formed to face each other and to be spaced apart along the longitudinal axis C, airflow passing through the slits may pass a central area of the capsule 232.

Referring to FIG. 4C, the functional substance accommodation member 230 may be completely separated from the holder 210, but a state in which the capsule 232 is inserted in the holder 210 may be maintained. In this case, when the functional substance is transferred, separation of the functional substance may be prevented compared to a case in which the functional substance accommodation member 230 is completely separated from the holder 210, and thus, unnecessary leakage of the functional substance may be prevented.

When the user bites one end of the stick 231 and makes an inhaling motion while the slits are formed in the capsule 232, external air may flow into the internal space of the holder 210 through an airflow hole (not shown) formed in the holder 210, and the air may flow into the capsule 232 through the slit formed by the second piercing member 222. Subsequently, the air may escape through the slit formed by the first piercing member 221 and reach the user, in which case the functional substance in the capsule 232 may also be transferred to the user. Due to eccentric positions of the slits, the air may pass a central part of the capsule 232, and a vortex behavior of the functional substance may be induced. The vortex behavior may induce smooth movement of the functional substance, and accordingly, discharge of the functional substance may be performed smoothly.

In an example, when the capsule 232 is disposed in the stick 231 in an unfixed state, air movement (airflow) generated due to an eccentric position of the slits may induce the rotation of the capsule 232. Through this, the discharge of the functional substance may be performed more smoothly.

The description of the above-described embodiments is only illustrative, and one of ordinary skill in the art will understand that various modifications and other equivalent embodiments are possible therefrom. Therefore, the true scope of protection of the present disclosure should be determined by the appended claims, and all differences within the equivalent scope of what is stated in the claims should be interpreted as being included in the scope of protection determined by the claims.

## Claims

1. An inhaler for inhaling a functional substance, the inhaler comprising:
a holder comprising an opening and an internal space that communicates with the opening;
a piercing member protruding toward the internal space; and
a functional substance accommodation member inserted from the opening into the internal space of the holder along a longitudinal axis of the holder and configured to accommodate the functional substance,
wherein the piercing member is configured to be movable between a first piercing position and a second piercing position, and wherein a cutting edge of the piercing member is closer to the longitudinal axis in the second piercing position than in the first piercing position.

2. The inhaler of claim 1, wherein the piercing member comprises:
a first piercing member; and
a second piercing member disposed spaced apart from the first piercing member along the longitudinal axis.

3. The inhaler of claim 2, wherein the first piercing member and the second piercing member are disposed to face each other about the longitudinal axis.

4. The inhaler of claim 2, wherein a cutting edge of the first piercing member and a cutting edge of the second piercing member are directed toward the longitudinal axis of the holder.

5. The inhaler of claim 1, wherein
the piercing member is configured to be capable of protruding in a direction perpendicular to the longitudinal axis,
the first piercing position is a state in which the piercing member returns to an initial position, and
the second piercing position is a state in which the piercing member protrudes toward the longitudinal axis.

6. The inhaler of claim 1, wherein
the piercing member is pivotally rotatably coupled to the holder,
the inhaler further comprises a stopping member in contact with the piercing member and configured to prevent a rotation of the piercing member, and
the stopping member is configured to prevent the piercing member from rotating toward the opening.

7. The inhaler of claim 6, wherein
the first piercing position is a state in which the cutting edge of the piercing member is rotated toward an opposite side of the opening, and
the second piercing position is a state in which the cutting edge of the piercing member is rotated toward the longitudinal axis.

8. The inhaler of claim 1, wherein a marker for indicating a length at which the functional substance accommodation member is inserted into the internal space is provided in at least one of the functional substance accommodation member or the holder.

9. The inhaler of claim 1, wherein the functional substance accommodation member comprises:
a stick insertable into the internal space of the holder; and
a capsule disposed in an inside of the stick and configured to accommodate the functional substance.
